# EUROPÄISCHE PATENTSCHRIFT

(11) **EP 0 631 794 B1**
(45) Veröffentlichungstag und Bekanntmachung des Hinweises auf die Patenterteilung: **02.09.1998**
(21) Anmeldenummer: 94109161.3
(22) Anmeldetag: 15.06.1994
(51) Int. Cl.: A61M 37/00

(54) **Vorrichtung zum Applizieren von Implantaten**
Device for administering implants
Dispositif pour administrer des implants

(30) Priorität: 23.06.1993 DE 4320754
(43) Veröffentlichungstag der Anmeldung: 04.01.1995
(73) Patentinhaber: HOECHST AKTIENGESELLSCHAFT, 65929 Frankfurt am Main (DE)
(72) Erfinder: Loos, Hans-Joachim, D-65462 Ginsheim-Gustavsburg (DE); Ziegert, Günter, D-65929 Frankfurt (DE); Pajunk, Horst, D-78187 Geisingen (DE); Pajunk, Heinrich, D-78187 Geisingen (DE)

(56) Entgegenhaltungen:
- EP-A- 0 304 700
- WO-A-92/15362
- DE-A- 3 802 158
- GB-A- 786 850
- US-A- 4 787 384

## Beschreibung

Gegenstand der Erfindung ist eine Vorrichtung zum Applizieren von Implantaten gemäß dem Oberbegriff des Anspruchs 1.

Vorrichtungen der genannten Art sind aus der GB-A-786850 bekannt. Nachteilig bei diesen Vorrichtungen ist, daß das stäbchenförmige Implantat (ein sogenanntes rod), das ein Medikament enthalten kann, beim Applizieren durch die Haltevorrichtung beschädigt werden kann.

Hier will die Erfindung Abhilfe schaffen. Die Aufgabe wird durch die Merkmale des kennzeichnenden Teils des Anspruchs 1.

Die Haltevorrichtung kann aus zwei gegenüber angeordneten, elastischen Bolzen mit je einem verrundeten Ende bestehen, das in den Stößelkanal hineinragt. Ferner kann die Haltevorrichtung aus zwei gegenüber angeordneten, federbelasteten Bolzen mit je einem verrundeten Ende bestehen, das in den Stößelkanal hineinragt. In einer weiteren Ausgestaltung kann die Haltevorrichtung aus mindestens einer, vorzugsweise zwei federbelasteten Kugeln bestehen, die in den Stößelkanal hineinragen.

Die Haltevorrichtung verhindert nicht nur das unbeabsichtigte Herausrutschen des Implantats aus der Applikationsvorrichtung, sondern gibt auch den Stößelkanal ohne Schädigung des Implantats frei.

Im folgenden wird die Erfindung an Hand von lediglich einen Ausführungsweg darstellenden Zeichnungen näher erläutert.

Es zeigt
- Figur 1: die Vorrichtung zum Applizieren von Implantaten geschnitten,
- Figur 2: die Einzelheit "Z" in einer alternativen Ausführungsform der Haltevorrichtung geschnitten und
- Figur 3: die Einzelheit "Z" in weiteren alternativen Ausführungsform der Haltevorrichtung geschnitten.

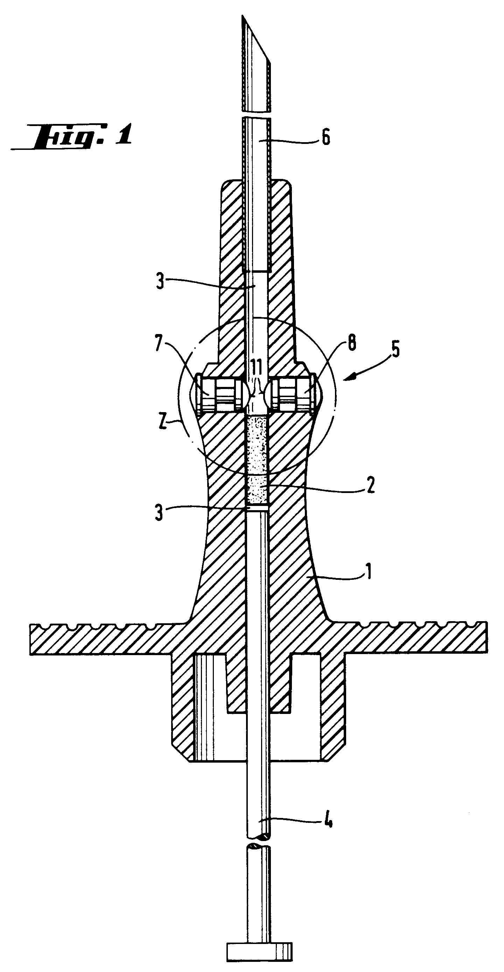

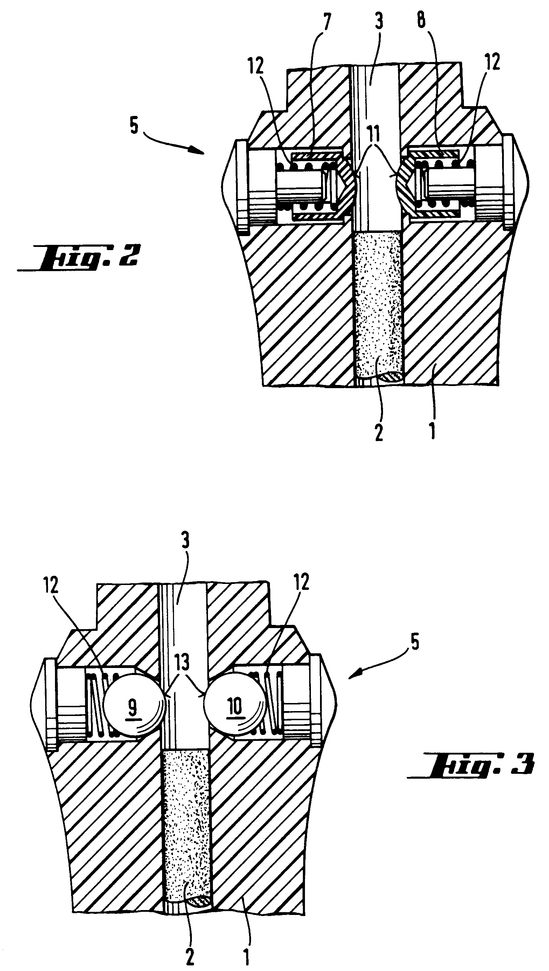

Die Vorrichtung zum Applizieren von Implantaten, sogenannten "rods" besteht aus einem Wirkstoffbehälter 1 mit Injektionskanüle 6 und Stößel 4. Der Stößel 4 ist in einem Stößelkanal 3 angeordnet, der auch das vorzugsweise stäbchenförmige Implantat 2 aufnimmt und somit als Implantatkammer dient. Der Stößelkanal 3 muß stetig in das Lumen der Kanüle 6 übergehen, damit Querschnittsänderungen auf dem Weg, den das Implantat beim Applizieren zurücklegt, vermieden werden. Damit das Implantat 2 nicht ungewollt den Wirkstoffbehälter 1 verlassen kann, ist im lumenseitigen Ende des Stößelkanals 3 eine Haltevorrichtung 5 für das Implantat 2 vorgesehen. Die Haltevorrichtung 5 ragt geringfügig in den Stößelkanal 3 hinein und bildet somit ein Hindernis für das Implantat 2. Um Deformationen des Implantats und/oder Abrieb von Implantat beim Applizieren zu vermeiden, sollte die Haltevorrichtung 5 so beschaffen bzw. angeordnet sein, daß sie unschädlich für das Implantat demselben ausweicht und somit den Weg freigibt. Die Haltevorrichtung 5 kann aus mindestens einem, üblicherweise zwei Bolzen 7, 8 mit je einem verrundeten Ende 11 bestehen. Die Bolzen 7, 8 können aus elastischem Material aus diversen Kautschukarten oder dergleichen hergestellt sein (Figur 1). Es eignen sich in gleicher Weise Bolzen 7, 8 aus nicht elastischem Material, die durch den Druck von Federn 12 in Position gehalten werden (Figur 2). Auch bei den starren Bolzen 7, 8 kann das in den Stößelkanal 3 ragende Ende 11 verrundet sein. Statt der Bolzen 7, 8 eignen sich gleichermaßen Kugeln, die mit ihren Kugelkappen 13 in den Stößelkanal 3 ragen (Figur 3). Die Kugeln 9, 10 können wie die Bolzen 7, 8 aus elastischem Material bestehen oder mit einer Feder 12 in ihrer Position gehalten werden. Federkraft, Elastizität und Oberflächenverrundungen sollten so beschaffen sein, daß das Implantat die Haltevorrichtung 5 ohne Deformation und/oder Abriebverluste passieren kann.

## Patentansprüche

1. Vorrichtung zum Applizieren von Implantaten, bestehend aus einem Wirkstoffbehälter (1) mit Injektionskanüle (6) und Stößel (4), wobei der Stößel (4) in einem Stößelkanal (3) angeordnet ist, der stetig in das Lumen der Kanüle (6) übergeht, und wobei im lumenseitigen Ende des Stößelkanals (3) eine Haltevorrichtung (5) für das Implantat (2) angeordnet ist, dadurch gekennzeichnet, daß die Haltevorrichtung (5) so beschaffen ist, daß sie dem Implantat (2) ausweichen und den Stößelkanal (3) freigeben kann.

2. Vorrichtung nach Anspruch 1, dadurch gekennzeichnet, daß die Haltevorrichtung (5) aus zwei gegenüber angeordneten, elastischen Bolzen (7, 8) mit je einem verrundeten Ende (11) besteht, das in den Stößelkanal (3) hineinragt.

3. Vorrichtung nach Anspruch 1, dadurch gekennzeichnet, daß die Haltevorrichtung (5) aus zwei gegenüber angeordneten, federbelasteten Bolzen (7, 8) mit je einem verrundeten Ende (11) besteht, das in den Stößelkanal (3) hineinragt.

4. Vorrichtung nach Anspruch 1, dadurch gekennzeichnet, daß die Haltevorrichtung (5) mindestens aus einer, vorzugsweise zwei gegenüber angeordneten, federbelasteten Kugeln (9, 10) besteht, die mit ihren Kugelkappen (13) in den Stößelkanal (3) hineinragen.

## Claims

1. A device for administering implants, comprising an active substance container (1) with injection cannula (6) and plunger (4), the plunger (4) being arranged in a plunger channel (3) which merges with continuity into the lumen of the cannula (6), and a holder device (5) for the implant (2) being arranged at the lumen-side end of the plunger channel (3), wherein the holder device (5) is designed such that it can move out of the way of the implant (2) and can clear the plunger channel (3).

2. The device as claimed in claim 1, wherein the holder device (5) comprises two elastic pins (7, 8) which are arranged opposite one another and which in each case have a rounded end (11) protruding into the plunger channel (3).

3. The device as claimed in claim 1, wherein the holder device (5) comprises two spring-loaded pins (7, 8) which are arranged opposite one another and which in each case have a rounded end (11) protruding into the plunger channel (3).

4. The device as claimed in claim 1, wherein the holder device (5) comprises at least one spring-loaded ball, preferably two spring-loaded balls (9, 10) arranged opposite one another and protruding into the plunger channel (3) via their spherical caps (13).

## Revendications

1. Dispositif d'application d'implants, constitué d'un récipient de principe actif (1) ayant une canule d'injection (6) et un coulisseau (4), le coulisseau (4) étant agencé dans un canal (3) qui se fond en permanence dans la lumière de la canule (6) et dans lequel, à l'extrémité du canal (3) du coulisseau côté lumière est agencé un dispositif de retenue (5) pour l'implant (2), caractérisé en ce que le dispositif de retenue (5) est tel qu'il puisse s'écarter de l'implant (2) et libérer le canal (3) du coulisseau.

2. Dispositif selon la revendication 1, caractérisé en ce que le dispositif de retenue (5) est constitué de deux chevilles élastiques (7, 8) agencées l'une en regard de l'autre, ayant chacune une extrémité arrondie (11) qui fait saillie dans le canal (3) du coulisseau.

3. Dispositif selon la revendication 1, caractérisé en ce que le dispositif de retenue (5) est constitué de deux chevilles (7, 8) agencées l'un en regard de l'autre et sollicitées par des ressorts, ayant chacune une extrémité arrondie (11) qui fait saillie dans le canal (3) du coulisseau.

4. Dispositif selon la revendication 1, caractérisé en ce que le dispositif de retenue (5) est constitué d'au moins une, de préférence deux billes agencées l'une en face de l'autre (9, 10) et sollicitées par des ressorts, qui font saillie par leurs coiffes sphériques (13) dans le canal (3) du coulisseau.
